# EUROPEAN PATENT APPLICATION

(11) **EP 4 052 693 A1**
(43) Date of publication of application: **07.09.2022**
(21) Application number: 21305262.4
(22) Date of filing: 04.03.2021
(51) Int. Cl.: A61K 6/56, A61K 6/78, A61K 6/853, A61K 6/856, A61K 6/873, A61K 6/876, C04B 28/18, C04B 111/00

(54) **HARDENED CALCIUM SILICATE-BASED DENTAL MATERIAL WITH IMPROVED MECHANICAL PROPERTIES**

(71) Applicant: Septodont ou Septodont SAS ou Specialites Septodont, 94100 Saint Maur des Fossés (FR)
(72) Inventor: DJOUDI, Mounir, 35740 Pacé (FR); RICHARD, Gilles, 91560 Crosne (FR); LEROUX, Esther, 77500 Chelles (FR)
(74) Representative: Icosa

(57) **Abstract**

The present invention relates to a hardened dental material comprising calcium silicate, calcium carbonate, at least one pozzolanic material and a compound of general formula mCaO·nSiO₂·pH₂O in which m and n each independently vary from 1 to 3 and p varies from 3 to 6. The invention further relates to a dental composition, to a method of preparing the hardened dental material of the invention, to a kit for preparing the hardened dental material, to a medical device comprising said kit and to the use of the hardened dental material for treating the crown of a tooth and/or the root of a tooth.

## Description

### FIELD OF INVENTION

The present invention relates to the field of dentistry. Especially, the invention relates to the provision of a hardened dental material with improved mechanical properties, especially an improved compressive strength.

### BACKGROUND OF INVENTION

Dental restoration aims at restoring of the integrity and morphology of teeth, including restoring the loss of mineralized substance due to carries or resulting from an external trauma. Direct restoration is performed by placing a malleable filling material into a prepared tooth, followed by the *in situ* setting of the material.

Ideally, the dental material should possess several properties, including adequate adhesive ability, insolubility, dimensional stability, biocompatibility, bioactivity and suitable mechanical properties. Various types of filling material are available, among which calcium silicate-based cements.

The implementation of the dental material often requires a first phase of preparation by the practitioner of the filling material, followed by a period of *in situ* hardening. This is especially the case when using a hydraulic dental cement, such as a calcium silicate-based cement, which has to be exposed to water, usually by mixing an anhydrous powder cement phase with a liquid aqueous phase, in order to initiate hardening.

The main parameters to be controlled when providing a dental calcium silicate-based cement comprise the handling properties, the setting time and the mechanical properties of the hardened material.

Regarding the handling properties, the texture of the filling material has to be creamy for a good handling by the practitioner. Further, the working time should be just sufficient to enable the preparation of the filling material and its placement where restoration is needed.

The setting time should ideally be relatively short. Indeed, a too long setting time would be uncomfortable for the patient, may lead to the washout of the restorative material by saliva and to the irritation of oral tissues.

The hardened restoration material should have mechanical properties similar to those of the teeth. Especially, the compressive strength has to be sufficient to avoid breakage of the restorative material and ensure its longevity.

European patents EP 2 555 740 and EP 1 531 779 disclose compositions suitable for restoring mineralized substance, especially in the dental field. Said compositions are obtained from the mixing of an aqueous liquid solution with a powder comprising calcium silicate, calcium carbonate, calcium oxide, and optionally zirconium oxide and/or at least one pigment. Selection of suitable ranges for each component and of suitable weight ratios between the aqueous liquid solution and the powder affords a hardened dental material presenting suitable mechanical properties. For instance, the compressive strength of the obtained hardened dental material is of around 100 to 200 MPa.

However, there still exists a demand of patients and practitioners for dental materials having higher mechanical properties, in order to improve their resistance to breakage and their longevity.

The inventors surprisingly evidenced that including at least one pozzolanic material in a known composition suitable as dental composition afforded a consequent increase, of the order of 14 to 30%, of the compressive strength of the obtained hardened dental material.

The use of pozzolanic materials is known with Portland cements in order to improve the properties thereof.

Nevertheless, to the inventors' knowledge, the use of pozzolanic materials for improving the mechanical properties, especially the compressive strength, of a hardened dental material based on calcium silicate was never disclosed.

### SUMMARY

The present invention relates to a hardened dental material comprising:
- from 5% to 65%, by weight with respect to the total weight of the material, of calcium silicate;
- from 1% to 30%, by weight with respect to the total weight of the material, of a mixture comprising calcium carbonate and at least one pozzolanic material;
- from more than 0% to 50%, preferably from 1% to 40% by weight with respect to the total weight of the material, of a compound of general formula mCaO·nSiO₂·pH₂O in which m and n each independently vary from 1 to 3 and p varies from 3 to 6.

According to an embodiment, the at least one pozzolanic material comprises, preferably consists of, silica fume.

According to an embodiment, the hardened dental material comprises:
- from 0.5% to 20%, of silica fume, by weight with respect to the total weight of the material, and
- from 0.5% to 20%, of calcium carbonate, by weight with respect to the total weight of the material.

According to an embodiment, the calcium silicate is pure tricalcium silicate.

According to an embodiment, the calcium silicate is a mixture of tricalcium silicate and dicalcium silicate, said mixture being such that it contains no more than 10% by weight of dicalcium silicate with respect to the total weight of the calcium silicates present in the composition.

According to an embodiment, the hardened dental material further comprises a setting accelerator, preferably calcium oxide or calcium chloride.

According to an embodiment, the hardened dental material further comprises a radiopacifier, preferably zirconium oxide or bismuth oxide.

According to an embodiment, the hardened dental material further comprises at least one pigment, preferably at least one iron oxide.

According to an embodiment, the hardened dental material further comprises at least one texturing agent.

The present invention further relates to a dental composition comprising:
- from 15% to 98% in weight of the total weight of the composition of calcium silicate;
- from 0.5% to 20 % in weight of the total weight of the composition of calcium carbonate, preferably 4%;
- from 0.5% to 20 % in weight of the total weight of the composition of at least one pozzolanic material, preferably 10%;
- optionally from 2% to 35% in weight of the total weight of the composition of a radiopacifier; and
- optionally one or more additive selected from setting accelerators, pigments, water reducing agents, texturing agents, pH stabilizing agents, surfactants, and fillers.

The present invention further relates to a method of manufacturing the hardened dental material according to the invention, characterized in that it comprises a mixing step of a solid phase consisting of a mixture of powders and of a liquid aqueous phase,
said solid phase comprising a dental composition according to the invention,
said liquid aqueous phase comprising calcium chloride, a polycarboxylate and water,
wherein the weight ratio of solid phase to liquid aqueous phase ranges from 2 to 5, preferably ranges from 2.5 to 4.0.

The present invention further relates to a kit for producing a hardened dental material, said kit comprising:
- a first container containing a powder phase comprising:
   - from 15% to 98% in weight of the total weight of the powder phase of calcium silicate;
   - from 0.5% to 20 % in weight of the total weight of the powder phase of calcium carbonate;
   - from 0.5% to 20 % in weight of the total weight of the powder phase of at least one pozzolanic material,
   - optionally from 2% to 35% in weight of the total weight of the powder phase of a radio-opacifier; and
   - optionally one or more additive selected from setting accelerators, pigments, water reducing agents, texturing agents, pH stabilizing agents, surfactants, and fillers; and
- a second container containing a liquid aqueous phase;
wherein the weight ratio of the powder phase present in the kit to the liquid aqueous phase present in the kit ranges from 2 to 5; preferably from 2.5 to 4.0.

According to an embodiment, the hardened dental material to be produced with the kit of the invention is a hardened material according to the invention.

The present invention further relates to a medical device comprising the kit according to the invention.

The present invention finally relates to the use of a hardened dental material according to the invention, for treating the crown of a tooth and/or the root of a tooth.

### DEFINITIONS

In the present invention, the following terms have the following meanings:
- **"About"** preceding a figure means plus or less 10% of the value of said figure.
- **"Additive"** refers to any substance added, preferably in a low amount, in a composition for improving its physicochemical properties depending on its use.
- **"Aqueous"** refers to any compound or composition comprising water and/or moisture.
- **"Calcium silicate"** refers to compounds that can be produced by reacting calcium oxide and silica in various ratios. According to one embodiment, the expression "calcium silicate" refers to compounds made of calcium and silicate, preferably selected from tricalcium silicate, dicalcium silicate or any mixtures thereof; more preferably tricalcium silicate C3S (of formula Ca₃SiO₅), dicalcium silicate C2S (of formula Ca₂SiO₄), or any mixtures thereof.
- **"Calcium silicate particle":** refers to an assembly comprising one or more calcium silicate compounds. The terms "calcium silicate particle" also include assemblies consisting of one or more calcium silicate compounds.
- **"Coarsely grinded particles"** refers to particles having a dio size ranging from more than 1.7 up to 5 µm; a dso size ranging from more than 8 up to 14 µm and a d₉₀ size ranging from more than 20 up to 40 µm; and a specific area ranging from 0.3 to 1.2 m²/g.
- **"Crushed particles"** refers to particles having a dio size ranging from more than 2 up to 6 µm; a dso size ranging from more than 17 up to 25 µm and a d₉₀ size ranging from more than 150 up to 330 µm; and a specific area of about 0.5 m²/g.
- **"Dental cement"** refers to any composition suitable for endodontic and/or restorative dentistry that acts as an adhesive to hold together the casting to the tooth structure.
- **"Dental composition"** refers to any formulation suitable for dental applications.
- **"Dual syringe"** refers to an injection system comprising or consisting of a mixing system and/or a mixing chamber, two cartridges and a plunger.
- **"d₁₀ size"** means that 10% of the particles have a mean diameter less than said value. According to one embodiment, the dio size is measured by laser diffraction.
- **"dso size"** means that 50% of the particles have a mean diameter less than said value. According to one embodiment, the dso size is measured by laser diffraction.
- **"d₉₀ size"** means that 90% of the particles have a mean diameter less than said value. According to one embodiment, the d₉₀ size is measured by laser diffraction.
- **"Hardened dental material"** refers to a material suitable for dental applications that is under a solid form. According to one embodiment, the expression "hardened dental material" refers to the material obtained after the hardening (or setting) of the dental composition of the invention. A "hardened dental filling material" especially refers to a hardened dental material suitable for filling dental restoration.
- **"Hydraulic cement"** refers to a cement able to self-harden when contacted with water.
- **"Medical device"** refers to any apparatus, material or object used alone or in combination, which may be used for diagnostic and/or therapeutic purposes.
- **"Micronized particles"** refers to particles having a dio size ranging from more than 0.7 up to 1.7 µm; a dso size ranging from more than 2.9 up to 8 µm and a d₉₀ size ranging from more than 6 up to 20 µm; and a specific area ranging from 0.8 to 3 m²/g.
- **"Pigment"** refers to any coloring chemical compound that may be natural or synthetic, mineral or organic.
- **"Pozzolanic material"** refers, according to ASTM C125, to a natural or artificial siliceous or siliceous and aluminous material which, in itself, possesses little or no cementitious value but which will, in finely divided form and in the presence of water, reacts chemically with calcium hydroxide at ordinary temperature to form compounds possessing cementitious properties. An artificial pozzolanic material may comprise or consisting of, for instance, an industrial by-product such as fly ash, silica fume from silicon smelting, highly reactive metakaolin, slag, burned organic matter residues rich in silica such as rice husk ash, calcined clay, or any mixture thereof. A natural pozzolanic material may comprise or consist of, for instance, volcanic ashes, pumices, zeolites, diatomaceous earths and any mixture thereof.
- **"Radiopacifier"** refers to a substance added to a material in order to make it opaque, especially to make it visible under X-ray imaging.
- **"Setting accelerator"** refers to any agent which reduces the setting time of a material when added to said material compared to the setting time of the same material without said agent.
- **"Setting time"** herein refers to the period of time needed for a dental composition of the invention to be totally hardened after its hydration. The setting time starts when placing the tested composition in defined conditions of temperature and hygrometry (typically water bath at 37°C). The setting time may be measured by several methods such as for example a Gillmore apparatus (Gillmore needle) or a Vicat apparatus (Vicat needle). For example; the setting time may be measured using a Gillmore apparatus: the material to be tested is placed into a mold which is introduced in a water bath at 37 °C, the setting of the material is assessed using a Gillmore needle of 400g, and the material is considered as being set when the needle leaves no trace on the surface of the mold. The setting time corresponds to the period of time between the placement of the molds into the water bath and the observed setting.
- **"Silica fume"** refers to an amorphous (non-crystalline) polymorph of silicon dioxide, silica. Silica fume is an ultrafine powder collected as a by-product of the silicon and ferrosilicon alloy production and consists of spherical particles with an average particle diameter of 150 nm. Silica fume is also known as microsilica, its CAS number is 69012-64-2 and its EINECS number is 273-761-1.
- **"Texturing agent"** refers to any compound which, when added to a substance, enhances the viscosity and the cohesion of said substance.
- **"Ultrafine particles":** refers to particles having a dio size of less than 0.7 µm; a dso size ranging from about 0.7 µm to 2.9 µm and a d₉₀ size ranging from about 2.0 µm to 7.0 µm; and a specific area measured by BET, ranging from about 3 to 11 m²/g. According to one embodiment, the d₁₀, d₅₀ and d₉₀ sizes are measured by laser diffraction. According to one embodiment, "ultrafine particles" also refers to particles having a dio size ranging from 0.4 µm to 0.9 µm, preferably ranging from 0.4 µm to 0.82 µm or 0.4 µm to 0.8 µm; a dso size ranging from 0.7 µm to 2.9 µm; and a d₉₀ size ranging from 1.3 µm to 7 µm. According to one embodiment, the ultrafine particles have a dio size ranging from 0.4 µm to 0.8 µm; a dso size ranging from 0.8 µm to 2.1 µm; and a d₉₀ size ranging from 1.4 µm to 7 µm; and a specific area measured by BET, ranging from about 3 to 11 m²/g.

Further, in the present invention, when referring to a range, the following is meant: "ranging from X to Y" means that X and Y are included in the range; "ranging from more than X, up to Y" means that X is not included in the range while Y is included in the range; and "less than X" means that the range includes X or lower values.

In the present invention, the term "comprise" and any variation thereof should be understood as open terms and as not excluding the presence of any further components (when it relates to a product) or of any further step (when it relates to a process). In specific embodiments, the term "comprise" and its variation may be understood as "consist essentially of', or even "consist of'.

### DETAILED DESCRIPTION

The invention relates to a hardened dental material comprising calcium silicate, at least one pozzolanic material, and a compound of general formula mCaO·nSiO₂·pH₂O in which m and n each independently vary from 1 to 3 and p varies from 3 to 6.

The hardened dental material of the invention preferably results from the hydration of a hydraulic cement comprising calcium silicate; preferably selected from tricalcium silicate, dicalcium silicate, Portland cement, mineral trioxide aggregate (MTA) and any combinations thereof; more preferably the composite material of the invention results from the hydration a hydraulic cement comprising tricalcium silicate. According to one embodiment, the hardened dental material of the invention results from the hydration of a hydraulic cement comprising calcium silicate particles.

According to an embodiment, the hardened dental material further comprises calcium carbonate.

According to an embodiment, the hardened dental material according to the invention comprises:
- from 5% to 65%, by weight with respect to the total weight of the material, of calcium silicate;
- from 1% to 20%, by weight with respect to the total weight of the material, of at least one pozzolanic material;
- from more than 0% to 50%, preferably from 1% to 40% by weight with respect to the total weight of the material, of a compound of general formula mCaO·nSiO₂·pH₂O in which m and n each independently vary from 1 to 3 and p varies from 3 to 6.

According to an embodiment, the hardened dental material according to the invention comprises:
- from 5% to 65%, by weight with respect to the total weight of the material, of calcium silicate;
- from 1% to 30%, by weight with respect to the total weight of the material, of a mixture comprising calcium carbonate and at least one pozzolanic material;
- from more than 0% to 50%, preferably from 1% to 40% by weight with respect to the total weight of the material, of a compound of general formula mCaO·nSiO₂·pH₂O in which m and n each independently vary from 1 to 3 and p varies from 3 to 6.

### Calcium silicate

According to one embodiment, the calcium silicate is selected from tricalcium silicate, dicalcium silicate or any mixtures thereof; preferably is tricalcium silicate.

According to one embodiment, tricalcium silicate is selected from compounds of formula Ca₃SiO₅ (also noted as "C3S") and of formula Ca₃Si₃O₉ (also called "calcium oxosilanediolate"), preferably C3S.

According to one embodiment, dicalcium silicate is the compound of formula Ca₂SiO₄ (also noted as "C2S").

According to one embodiment, the calcium silicate is pure tricalcium silicate.

According to one embodiment, the calcium silicate is a mixture comprising or consisting essentially of tricalcium silicate and dicalcium silicate. Advantageously, this mixture is such that it comprises no more than 10% by weight of dicalcium silicate with respect to the total weight of the calcium silicates present in the composition, typically dicalcium silicate and tricalcium silicate. According to a preferred embodiment, the mixture comprises or consists of 0% to 10% dicalcium silicate and 90% to 100% tricalcium silicate, by weight with respect to the total weight of the calcium silicates. The term "consists of' means in particular that the mixture does not contain any calcium silicate other than dicalcium silicate and tricalcium silicate.

In an embodiment, the calcium silicate is in the form of calcium silicate particles.

In an embodiment, at least part of the calcium silicate, preferably all calcium silicate, is in the form of ultrafine calcium silicate particles. In said embodiment, the ultrafine particles of calcium silicate preferably have:
a dio size ranging from 0.4 µm to 0.9 µm, preferably ranging from 0.4 µm to 0.82 µm, more preferably ranging from 0.4 µm to 0.8 µm, even more preferably from 0.4 µm to 0.7 µm;
a dso size ranging from 0.7 µm to 2.9 µm, preferably ranging from 0.8 µm to 2.5 µm, more preferably ranging from 0.8 µm to 2.1, even more preferably from 1 µm to 2.1 µm; and
a d₉₀ size ranging from 1.3 µm to 7.0 µm, preferably ranging from 1.4 µm to 7 µm, more preferably ranging from 1.5 µm to 7 µm, even more preferably from 2 µm to 5 µm; wherein the d₁₀, d₅₀ and d₉₀ sizes are measured by laser diffraction.

In an embodiment, at least part of the calcium silicate, preferably all calcium silicate, is in the form of crushed calcium silicate particles. In said embodiment, the crushed calcium silicate particles preferably have:
a dio size ranging from more than 2 up to 6 µm;
a dso size ranging from more than 17 up to 25 µm; and
a d₉₀ size ranging from more than 150 up to 330 µm.

In an embodiment, at least part of the calcium silicate, preferably all calcium silicate, is in the form of coarsely grinded calcium silicate particles. In said embodiment, the coarsely grinded calcium silicate particles preferably have:
a d10 size ranging from more than 1.7 up to 5 µm;
a d50 size ranging from more than 8 up to 14 µm, and
a d90 size ranging from more than 20 up to 40 µm.

In an embodiment, at least part of the calcium silicate, preferably all calcium silicate, is in the form of micronized calcium silicate particles. In said embodiment, the micronized particles of calcium silicate preferably have:
a dio size ranging from more than 0.7 up to 1.7 µm;
a dso size ranging from more than 2.9 up to 8 µm,
and a d₉₀ size ranging from more than 6 up to 20 µm.

In one embodiment, the calcium silicate may be the calcium silicate present in a Portland cement. In another embodiment, the calcium silicate may be the calcium silicate present in mineral trioxide aggregate (MTA).

### Calcium Carbonate

The hardened dental material further comprises calcium carbonate CaCO₃, which is in the form of a white or roughly white powder practically insoluble in water.

In an embodiment, at least part of the calcium carbonate of the hardened dental material is in the form of calcium carbonate particles.

According to one embodiment, the calcium carbonate particles have a dio granulometry ranging from 1 nm to 500 nm, preferably a dio granulometry of 440 nm.

According to one embodiment, the calcium carbonate particles have a dso granulometry ranging from 1 nm to 1500 nm, preferably a dso granulometry of 1100 nm.

According to one embodiment, the calcium carbonate particles have a d₉₀ granulometry ranging from 1 nm to 5 000 nm, preferably a d₉₀ granulometry of 3550 nm.

According to the invention, the granulometry may be measured by a Beckman-Coulter LS230 granulometer appliance with SVM module.

According to one embodiment, the amount of calcium carbonate, preferably of calcium carbonate particles, in the hardened dental material of the invention ranges from more than 0% wt. to 20% wt., preferably from 1% wt. to 15% wt., more preferably from 1% wt. to 7% wt. by the total weight of said hardened dental material. According to one embodiment, the amount of the calcium carbonate is of 1, 2, 3, 4, 5, 6, 7, 8, 9, 10, 11, 12, 13, 14, 15, 16, 17, 18, 19 or 20% wt., by the total weight of said hardened dental material.

### Pozzolanic material

The hardened dental material further comprises at least one pozzolanic material.

In an embodiment, the at least one pozzolanic material is selected from the group consisting of fly ash, silica fume, metakaolin, slag and rice husk ash. Preferably, the at least one pozzolanic material is silica fume.

According to one embodiment, the amount of pozzolanic material in the hardened dental material of the invention ranges from more than 0% wt. to 20% wt., preferably from 1% wt. to 15% wt., more preferably from 1% wt. to 7% wt. by the total weight of said hardened dental material. According to one embodiment, the amount of the pozzolanic material in the composite material of the invention is 1, 2, 3, 4, 5, 6, 7, 8, 9, 10, 11, 12, 13, 14, 15, 16, 17, 18, 19 or 20% wt., by the total weight of said hardened dental material.

In an embodiment, the pozzolanic material and the calcium carbonate are present as a mixture of pozzolanic material and calcium carbonate. In a preferred embodiment, the mixture of pozzolanic material and calcium carbonate represents from more than 0% wt. to 30% wt., preferably from 1% wt to 20% wt, more preferably from 1% wt. to 15% wt. by the total weight of said hardened dental material.

In an embodiment, the mixture of pozzolanic material and calcium carbonate comprises from 0.5% to 20% of pozzolanic material and from 0.5% to 20% of calcium carbonate, in weight with respect to the total weight of the hardened dental material.

### Compound of general formula mCaO·nSiO₂·pH₂O

The hardened dental material further comprises a compound of general formula mCaO·nSiO₂·pH₂O.

The compound of general formula mCaO·nSiO₂·pH₂O, is generally referred to as CSH for Calcium Silicate Hydrates. CSH may be obtained by reacting calcium silicate with water.

n, m and p are integers. n and m, each independently, vary from 1 to 3, and p varies from 3 to 6. In an embodiment, n is 1, 2 or 3. In an embodiment, m is 1, 2 or 3. In an embodiment, p is 3, 4, 5 or 6. In an embodiment, m is 3, n is 2 and p is 3.

In the case of tricalcium silicate, CSH is a hydrate that forms by heterogeneous germination on the surface of the tricalcium silicate crystals and develops by aggregation of nanocrystals in order to cover the entire surface of the calcium silicate crystals.

The hardened dental material according to the invention unexpectedly presents interesting mechanical properties. Actually, replacing at least part of the calcium carbonate of a known hardened dental material with at least one pozzolanic material unexpectedly afforded a 14 to 30% increase of the compressive strength of said material.

The compressive strength is advantageously measured 24 h or72 h after mixing a powder phase (or a hydraulic cement) and an aqueous phase to form the hardened dental material. Typically, the compressive strength of this hardened dental material 24 h after mixing the powder phase and the liquid aqueous phase has been determined to range from 250 MPa to 330 MPa. Typically, the compressive strength of this hardened dental material 72 h after mixing the powder phase and the liquid aqueous phase has been determined to range from 270 MPa to 370 MPa.

The compressive strength may be measured with reference to ISO 9917-1: 2007. The test tubes are prepared after mixing, using cylindrical Teflon moulds 4 mm in diameter and 6 mm high. In filling the moulds care is taken to eliminate the air bubbles from the paste, stored in an incubator for 15 minutes at 37oC and 100% relative humidity for the required setting time. The test tubes are then removed from the mould and stored under distilled water at 37oC for the wanted setting time. The samples are thus preserved under conditions close to their future conditions of clinical use. The compressive strength of the test tubes is measured on five occasions, i.e. 1 hour, 24 hours, 72 hours, 7 days and 28 days, by means of a Universal press (model 2/M, MTS Systems, 1400 Eden Prairie, Minneapolis, USA) with a speed of movement of 0.5 mm per minute.

The invention further relates to a dental composition comprising calcium silicate, calcium carbonate and at least one pozzolanic material.

The dental composition according to the invention is suitable for preparing a hardened dental material according to the invention. Preferably, in order to prepare the hardened dental material according to the invention, the dental composition according to the invention is mixed with an aqueous phase, preferably a liquid aqueous phase.

According to one embodiment, the dental composition according to the invention does not comprise any aluminate, such as calcium aluminate. According to one embodiment, the dental composition according to the invention does not comprise any halogen or halogenated compounds, such as for example fluoride. According to one embodiment, the dental composition according to the invention does not comprise any phosphates such as for example calcium phosphate. According to one embodiment, the dental composition according to the invention does not comprise any porous compounds. According to one embodiment, the dental composition according to the invention does not comprise any porous fillers and/or porous fibers.

The dental composition or the hardened dental material of the invention is intended to be in prolonged contact (more than 30 days) with dental tissues. The dental composition or the hardened dental material of the invention is particularly suitable for restoring decay lesions in dental crowns.

According to one embodiment, the dental composition according to the invention further comprises at least one additive, preferably selected from the group consisting of radiopacifiers, setting accelerators, pigments, water reducing agents, texturing agents, pH stabilizing agents, surfactants, fillers, and any mixtures thereof.

According to one embodiment, the at least one additive is comprised in the dental composition in an amount ranging from 0% to 60% in weight to the total weight of the composition; preferably from 2% to 50%; more preferably from 2% to 35%.

According to one embodiment, the dental composition comprises at least one radiopacifier. The radiopacifier is preferably selected from the group consisting of zirconium oxide, bismuth oxide, cerium oxide, barium sulphate, calcium tungstate, titanate dioxide, ytterbium oxide and mixtures thereof. In a specific embodiment, the radiopacifier is zirconium oxide or bismuth oxide, in particular zirconium oxide.

According to one embodiment, the dental composition comprises from 0 to 40% of radiopacifier in weight to the total weight of said composition; preferably from 2 to 35%, from 5 to 35%, preferably 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35%.

According to one embodiment, the dental composition comprises at least one setting accelerator. The setting accelerator is preferably selected from the group consisting of calcium carbonate, calcium oxide, calcium phosphate, sodium bicarbonate, calcium lactate, calcium chloride and any mixtures thereof, preferably calcium carbonate, calcium oxide or mixtures thereof. According to one embodiment, the setting accelerator is calcium oxide or calcium chloride.

According to one embodiment, the dental composition comprises from 0 to 25% of setting accelerator in weight to the total weight of said composition; preferably from 4 to 20%, preferably from 4 to 15%, preferably 1, 2, 3, 4, 5, 6, 7, 8, 9, 10, 11, 12, 13, 14, 15, 16, 17, 18, 19 or 20%.

According to one embodiment, the dental composition comprises at least one pigment. The pigment is preferably selected from iron oxides. One skilled in the art is able to select suitable mixtures of pigments so that the composition has the expected color. For instance, the iron oxide may be selected from the group consisting of yellow, red and brown iron oxides.

According to one embodiment, the dental composition comprises from 0 to 1.5% of pigment in weight to the total weight of said composition; preferably from 0.5% to 1%.

According to one embodiment, the dental composition comprises at least one water-reducing agent. The water-reducing agent is preferably selected from the group consisting of glenium, polynaphthalene sulfonate and modified polycarboxylate.

According to one embodiment, the dental composition comprises at least one texturing agent. The texturing agent is preferably selected from the group consisting of silica, povidone (also named polyvinylpyrrolidone), cellulose or derivatives thereof such as methylcellulose, hydroxypropylcellulose and hydroxyethylcellulose, polymers such as acrylamide/sodium acryloyldimethyltaurate copolymer isohexadecane and hydroxyethyl acrylate/sodium acryloyl dimethyl taurate copolymer, fumed silica (hydrophilic and/or hydrophobic), xanthan gum, and any mixtures thereof.

According to one embodiment, the dental composition comprises at least one pH stabilizing agent. According to one embodiment, the pH stabilizing agent is a mineral acid or an organic acid.

According to one embodiment, the dental composition comprises at least one surfactant. According to one embodiment, the surfactant is a polysorbate.

According to one embodiment, the dental composition comprises at least one filler. According to one embodiment, the filler is a mineral filler.

The dental composition may further comprise any other component or additive known in the art and suitable for improving any of its properties, such as its mechanical properties, its setting time, its texture and/or its appearance. The nature and amount of the additives to be added to the dental composition may be easily determined by one of ordinary skill in the art on the basis of the desired properties of the dental composition and/or of the hardened dental material to be obtained.

In an embodiment, the dental composition according to the invention comprises:
- from 15% to 98% in weight of the total weight of the composition of calcium silicate;
- from 0.5% to 20 %, preferably 4% in weight of the total weight of the composition of calcium carbonate;
- from 0.5% to 20 %, preferably 10%, in weight of the total weight of the composition of at least one pozzolanic material,
- optionally from 2% to 35% in weight of the total weight of the composition of a radiopacifier; and
- optionally one or more additive selected from setting accelerators, pigments, water reducing agents, texturing agents, pH stabilizing agents, surfactants, and fillers.

In a preferred embodiment, the dental composition is anhydrous or water-free. Indeed, in presence of water, calcium silicate begins hardening.

The invention further relates to a method of manufacturing the hardened dental material according to the invention. Said method is characterized in that it comprises a mixing step of a solid phase consisting of a mixture of powders and of a liquid aqueous phase.

The solid phase preferably comprises a mixture of powders of:
- calcium silicate,
- calcium carbonate,
- at least one pozzolanic material,
- and optionally zirconium oxide and/or at least one pigment.

In an embodiment, the solid phase is a dental composition according to the invention.

According to one embodiment, the liquid aqueous phase comprises water, preferably purified water.

According to one embodiment, the liquid aqueous phase consists in water. In another embodiment, the liquid aqueous phase is an aqueous solution.

According to one embodiment, the liquid aqueous phase comprises from 10 to 100% of water, in weight to the total weight of said liquid aqueous phase, preferably from 20% to 90%, preferably from 30% to 90%, preferably from 35% to 85%. According to one embodiment, the liquid phase comprises from 50% to 90% of water in weight to the total weight of said liquid phase, preferably from 60% to 90%, more preferably from 60% to 85%, more preferably from 65% to 85 %.

According to one embodiment, the liquid aqueous phase comprises at least one additive, wherein the additive is preferably selected from setting accelerators and water reducing agents. According to one embodiment, the liquid aqueous phase comprises one or more additives selected from setting accelerators (such as calcium chloride), water reducing agents (such as modified polycarboxylate, glenium, polynaphthalene sulfonate or mixtures thereof) and mixtures thereof. The liquid aqueous phase preferably comprises calcium chloride, a polycarboxylate and water.

According to one embodiment, the liquid aqueous phase comprises at least one additive in an amount ranging from 0% to 40% in weight to the total weight of the liquid aqueous phase; preferably from 10% to 35%; more preferably from 15% to 35%.

The weight ratio of the solid phase to the liquid aqueous phase to be mixed advantageously ranges from 2 to 5, preferably from 2.0 to 4.5 or from 2.5 to 4.0. According to one embodiment, the weight ratio of the powder phase to the liquid aqueous phase is 2,0; 2,1; 2,2; 2,3; 2,4; 2,5; 2,6; 2,7; 2,8; 2,9; 3.0; 3.1; 3.2; 3,3; 3,4; 3,5; 3,6; 3,7; 3,8; 3,9; 4.0; 4,1; 4,2; 4,3; 4,4 or 4,5.

Incorporating a pozzolanic material, in particular silica fume, in the dental composition of the invention allows using less aqueous phase for manufacturing the hardened material according to the invention. Without wishing to be bound by any theory, the Inventors believe that this advantage may be due to a fluidifying effect of the pozzolanic material after the mixing step is performed.

The mixing step may be performed by any suitable method known in the art. According to one embodiment, the mixing step is implemented by a vibration mixer.

According to one embodiment, the method for manufacturing the hardened dental material comprises at least one mixing step by vibration of the powder phase with the liquid aqueous phase.

According to one embodiment, the mixing step is implemented with a vibration frequency ranging from 1 rpm to 15 000 rpm; preferably ranging from 1 rpm to 10 000 rpm, preferably ranging from 1000 rpm to 6 000 rpm; more preferably ranging from 3 000 rpm to 5 000 rpm. According to one embodiment, the mixing step is implemented with a vibration frequency of about 1, 100, 200, 300, 400, 500, 600, 700, 800, 900, 1 000, 2 000, 3 000, 4 000, 5 000, 6 000, 7 000, 8 000, 9 000 or 10 000 rpm. According to another preferred embodiment, the mixing step is implemented with a vibration frequency ranging from 1 rpm to 15 000 rpm, preferably from 5 000 rpm to 15 000 rpm, more preferably from 10 000 rpm to 15 000 rpm. According to one embodiment, the mixing step is implemented with a vibration frequency of about 10 000 rpm, 11 000 rpm, 12 000 rpm, 13 000 rpm, 14 000 rpm or 15 000 rpm.

According to one embodiment, the mixing step by vibration is implemented during a vibration time ranging from 1 s to 3600 s; preferably from 1 s to 60 s; more preferably during 30 s. According to one embodiment, the mixing step by vibration is implemented during a vibration time of 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 s.

The invention further relates to a use of a hardened dental material according to the invention in the dental field, as a restorative and/or filling material.

The use of the hardened dental material of the invention may be for treating the crown of a tooth, for example temporary enamel restoration, permanent dentin restoration, deep or large carious lesions restoration, deep cervical or radicular lesions restoration, pulp capping or pulpotomy; and/or the root of a tooth, such as for example root and furcation perforations, internal/external resorptions, apexification or retrograde surgical filling.

The invention thus further relates to a hardened dental material of the invention for use for treating the crown of a tooth, for example enamel restoration, permanent dentin restoration, deep or large carious lesions restoration, deep cervical or radicular lesions restoration, pulp capping or pulpotomy; and/or the root of a tooth, such as for example root and furcation perforations, internal/external resorptions, apexification or retrograde surgical filling.

The invention also relates to a method for treating the crown of a tooth, for example temporary enamel restoration, permanent dentin restoration, deep or large carious lesions restoration, deep cervical or radicular lesions restoration, pulp capping or pulpotomy; and/or the root of a tooth, such as for example root and furcation perforations, internal/external resorptions, apexification or retrograde surgical filling; in a subject in need thereof, comprising the use of a hardened dental material according to the invention.

Another object of the present invention relates to a method for treating the crown of a tooth, for example temporary enamel restoration, permanent dentin restoration, deep or large carious lesions restoration, deep cervical or radicular lesions restoration, pulp capping or pulpotomy; and/or the root of a tooth, such as for example root and furcation perforations, internal/external resorptions, apexification or retrograde surgical filling; in a subject in need thereof, comprising the use of a composite material of the invention as defined above.

According to one embodiment, the composite materials of the invention may be used in treating a bone and/or dental disorder or disease in a subject in need thereof. According to one embodiment, the present invention refers to the use of the composite materials of the invention for treating a bone and/or dental disorder or disease in a subject in need thereof. According to one embodiment, the present invention refers to a method for treating a bone and/or dental disorder or disease in a subject in need thereof by using the composite materials of the invention.

The invention further relates to a kit for producing a hardened dental material, said kit comprising:
- a first container containing a powder phase comprising:
   - from 15% to 98% in weight of the total weight of the powder phase of calcium silicate;
   - from 0.5% to 20 % in weight of the total weight of the powder phase of calcium carbonate;
   - from 0.5% to 20 % in weight of the total weight of the powder phase of at least one pozzolanic material,
   - optionally from 2% to 35% in weight of the total weight of the powder phase of a radiopacifier; and
   - optionally one or more additive selected from setting accelerators, pigments, water reducing agents, texturing agents, pH stabilizing agents, surfactants, and fillers; and
- a second container containing a liquid aqueous phase;
wherein the weight ratio of the powder phase present in the kit to the liquid phase present in the kit ranges from 2 to 5; preferably from 2.5 to 4.0.

The liquid aqueous phase is as detailed above.

The kit of the invention is particularly suitable for manufacturing a hardened dental material according to the invention.

In a preferred embodiment the powder phase is anhydrous or water-free. Indeed, in presence of water, calcium silicate begins hardening. Therefore, it is important that the powder phase remains free of water during storage to avoid its undesirable setting at this stage.

The invention finally relates to a medical device comprising the kit according to the invention.

In one embodiment, the medical device is an injection system, preferably a syringe, comprising a composition obtained by the mixing of the powder phase and the liquid aqueous phase described above. In one embodiment, the medical device is an injection system, preferably a syringe, comprising the kit powder-liquid described above.

In another embodiment, the medical device is an injection system, preferably a syringe, comprising the dental composition of the invention. In a specific embodiment, the syringe is a dual syringe, one compartment comprising the dental composition of the invention and the second compartment comprising a liquid aqueous phase.

### BRIEF DESCRIPTION OF THE DRAWINGS

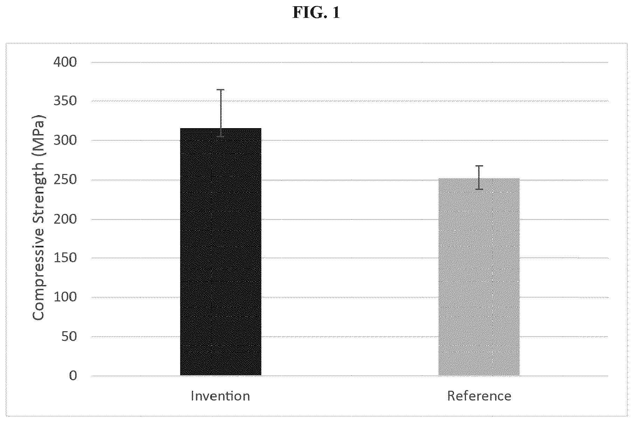

**Figure 1** is a graph comparing the measured compressive strength values at 72h for a hardened material according to the inventions and a reference hardened material not comprising silica fume.

### EXAMPLES

The present invention is further illustrated by the following examples, which are provided as illustrative, and not limitative, of the present invention.

### Example 1: Compressive strength of a hardened dental material of the invention at 24h

### Materials and Methods

The compressive strength of a hardened dental material comprising calcium silicate and a mixture of calcium carbonate and silica fume according to the invention was measured at 24h and compared to that of the reference material comprising calcium silicate and calcium carbonate, in absence of silica fume.

The tested hardened material is obtained by mixing 162 µL of water with a powder mixture comprising calcium silicate, fume silica and calcium carbonate.

The reference hardened material is obtained by mixing 173 µL of water with a powder mixture comprising calcium silicate and calcium carbonate.

Silica fume was 53 micrometers sieved.

The powder mixtures for both tested and reference hardened materials are detailed in table 1.

| **Component** | **Reference material (% w/w)** | **Hardened Material according to the invention (% w/w)** |
|---|---|---|
| **Tricalcium silicate** | 80.75 | 80.75 |
| **Silica fume** | - | 10 |
| **Calcium oxide** | 0.25 | 0.25 |
| **Calcium carbonate** | 14 | 4 |
| **Zirconium Oxide** | 5 | 5 |
| **Total** | 100 | 100 |

The compressive strength was measured for 7 samples of tested hardened material and for 8 samples of reference hardened material.

### Results

The measured compressive strengths for the different samples are presented in table 2.

**Table 2**

| **Compressive strength at 24h** | | | |
|---|---|---|---|
| **Hardened material according to the invention** | | **Reference material** | |
| **Sample n°** | **Compressive strength (Mpa)** | **Sample n°** | **Compressive strength (Mpa)** |
| 1 | 287,62 | 1 | 256,1 |
| 2 | 307,04 | 2 | 243,7 |
| 3 | 313,94 | 3 | 266,2 |
| 4 | 268,24 | 4 | 261,3 |
| 5 | 255,34 | 5 | 268,6 |
| 6 | 296,83 | 6 | 259,3 |
| 7 | 327,05 | 7 | 265,2 |
| | | 8 | 238,9 |
| **Mean value** | 293,72 | **Mean value** | 257,41 |

After 24h, the compressive strength of the hardened material according to the invention is increased of 14%.

The obtained results were further analyzed with a 2-sample Student t test, which confirmed that the compressive strength mean value obtained for the hardened material according to the invention is significantly higher than compressive strength mean value obtained for the reference material.

### Example 2: Compressive strength of a hardened dental material of the invention at 72h

### Materials and Methods

The compressive strength of a hardened dental material comprising calcium silicate and a mixture of calcium carbonate and silica fume according to the invention was measured at 72h and compared to that of the reference material comprising calcium silicate and calcium carbonate, in absence of silica fume.

The tested hardened material and reference hardened material are obtained as detailed in example 1 above.

Silica fume was 53 micrometers sieved.

The compressive strength was measured for 6 samples of tested hardened material and for 7 samples of reference hardened material.

### Results

The measured compressive strengths for the different samples are presented in table 3 and in figure 1.

**Table 3**

| **Compressive strength at 72h** | | | |
|---|---|---|---|
| **Hardened material according to the invention** | | **Reference material** | |
| **Sample n°** | **Compressive strength (Mpa)** | **Sample n°** | **Compressive strength (Mpa)** |
| 1 | 307,83 | 1 | 229,1 |
| 2 | 307,57 | 2 | 258,1 |
| 3 | 269,72 | 3 | 259,2 |
| 4 | 304,48 | 4 | 244,5 |
| 5 | 365,12 | 5 | 264,4 |
| 6 | 339,85 | 6 | 237,5 |
| | | 7 | 267,8 |
| **Mean value** | 315,76 | **Mean value** | 251,5 |

After 72h, the compressive strength of the hardened material according to the invention is increased of more than 25%.

The obtained results were further analyzed with a 2-sample Student t test, which confirmed that the compressive strength mean value obtained for the hardened material according to the invention is significantly higher than compressive strength mean value obtained for the reference material.

### Conclusion of the examples

The mechanical properties, in particular the compressive strength, of the hardened material according to the invention are significantly higher that the mechanical properties, in particular the compressive strength, of the reference hardened material not comprising silica fume. Consequently, the addition of a pozzolanic material such as silica fume confers increased mechanical properties to the hardened dental material.

The increase of compressive strength is higher at 72h than at 24h, suggesting that the long-term mechanical properties of the hardened material comprising the pozzolanic material will be at least as good as, if not higher, the mechanical properties at 72h.

Thus, the hardened material according to the invention presents improved mechanical properties when compared to the reference material, and said properties even improve over time.

## Claims

1. A hardened dental material comprising:
- from 5% to 65%, by weight with respect to the total weight of the material, of calcium silicate;
- from 1% to 30%, by weight with respect to the total weight of the material, of a mixture comprising calcium carbonate and at least one pozzolanic material;
- from more than 0% to 50%, preferably from 1% to 40% by weight with respect to the total weight of the material, of a compound of general formula mCaO·nSiO₂·pH₂O in which m and n each independently vary from 1 to 3 and p varies from 3 to 6.

2. The hardened dental material according to claim **1,** wherein the at least one pozzolanic material comprises, preferably consists of, silica fume.

3. The hardened dental material according to claim **2,** wherein the material comprises:
- from 0.5% to 20%, of silica fume, by weight with respect to the total weight of the material, and
- from 0.5% to 20%, of calcium carbonate, by weight with respect to the total weight of the material.

4. The hardened dental material according to any one of claims **1** to **3,** wherein the calcium silicate is pure tricalcium silicate.

5. The hardened dental material according to any one of claims **1** to **3,** wherein the calcium silicate is a mixture of tricalcium silicate and dicalcium silicate, said mixture being such that it contains no more than 10% by weight of dicalcium silicate with respect to the total weight of the calcium silicates present in the composition.

6. The hardened dental material according to any one of claims **1** to **5,** further comprising a setting accelerator, preferably calcium oxide or calcium chloride.

7. The hardened dental material according to any one of claims **1** to **6,** further comprising a radiopacifier, preferably zirconium oxide or bismuth oxide.

8. The hardened dental material according to any one of claims **1** to **7,** further comprising at least one pigment, preferably at least one iron oxide.

9. The hardened dental material according to any one of claims **1** to **8,** further comprising at least one texturing agent.

10. A dental composition comprising:
- from 15% to 98% in weight of the total weight of the composition of calcium silicate;
- from 0.5% to 20 % in weight of the total weight of the composition of calcium carbonate, preferably 4%;
- from 0.5% to 20 % in weight of the total weight of the composition of at least one pozzolanic material, preferably 10%;
- optionally from 2% to 35% in weight of the total weight of the composition of a radiopacifier; and
- optionally one or more additive selected from setting accelerators, pigments, water reducing agents, texturing agents, pH stabilizing agents, surfactants, and fillers.

11. A method of manufacturing the hardened dental material according to any one of claims **1** to **9, characterized in that** it comprises a mixing step of a solid phase consisting of a mixture of powders and of a liquid aqueous phase,
said solid phase comprising a dental composition according to claim **10,**
said liquid aqueous phase comprising calcium chloride, a polycarboxylate and water,
wherein the weight ratio of solid phase to liquid aqueous phase ranges from 2 to 5, preferably ranges from 2.5 to 4.0.

12. A kit for producing a hardened dental material, said kit comprising:
- a first container containing a powder phase comprising:
• from 15% to 98% in weight of the total weight of the powder phase of calcium silicate;
• from 0.5% to 20 % in weight of the total weight of the powder phase of calcium carbonate;
• from 0.5% to 20 % in weight of the total weight of the powder phase of at least one pozzolanic material,
• optionally from 2% to 35% in weight of the total weight of the powder phase of a radio-opacifier; and
• optionally one or more additive selected from setting accelerators, pigments, water reducing agents, texturing agents, pH stabilizing agents, surfactants, and fillers; and
- a second container containing a liquid aqueous phase;
wherein the weight ratio of the powder phase present in the kit to the liquid aqueous phase present in the kit ranges from 2 to 5; preferably from 2.5 to 4.0.

13. A kit for producing a hardened dental material according to claim **12,** wherein the hardened dental material to be produced is according to anyone of claims **1** to **11.**

14. A medical device comprising the kit according to claim **12.**

15. A use of a hardened dental material according to anyone of claims **1** to **11,** for treating the crown of a tooth and/or the root of a tooth.
